# EUROPEAN PATENT APPLICATION

(11) **EP 2 463 271 A1**
(43) Date of publication of application: **13.06.2012**
(21) Application number: 11460060.4
(22) Date of filing: 09.12.2011
(51) Int. Cl.: C07C 263/10, C07C 265/14, B01J 19/00

(54) **A method for producing toluene diisocyanate (TDI) by means of toluene diamine (TDA) phosgenation reaction in the gaseous phase and a device for obtaining toluene diisocyanate (TDI) by means of toluene diamine (TDA) phosgenation reaction in the gaseous phase**

(30) Priority: 10.12.2010 PL 39321610
(71) Applicant: Zaklady Chemiczne ZACHEM S.A., 85-825 Bydgoszcz (PL); POLITECHNIKA WARSZAWSKA, 00-661 Warszawa (PL); Instytut Chemii Przemyslowej Im. Prof Ignacego Moscickiego, 01-793 Warszawa (PL)
(72) Inventor: Slawatycki, Arkadiusz, 85-861 Bydgoszcz (PL); Chrupala, Wojciech, 85-704 Bydgoszcz (PL); Lachmajer, Jerzy, 85-149 Bydgoszcz (PL); Ruczynski, Lech, 85-565 Bydgoszcz (PL); Wojcik, Lucjan, 86-801 Niemcz (PL); Stuczynski, Jacek, 85-801 Bydgoszcz (PL); Baldyga, Jerzy, 05-540 Zalesie Gorne (PL); Jasinska, Magdalena, 02-920 Warszawa (PL); Szarlik, Stefan, 05-082 Blizne Laszczynskiego (PL); Wojcik, Wlodzimierz, 01-494 Warszawa (PL); Dyczewski, Michal, 05-420 Jozefow (PL)
(74) Representative: Twardowska, Aleksandra

(57) **Abstract**

The invention concerns a method for producing toluene diisocyanate (TDI) by means of the toluene diamine (TDA) phosgenation reaction in the gaseous phase and a device for obtaining toluene diisocyanate (TDI) by means of the toluene diamine (TDA) phosgenation reaction in the gaseous phase
The method for producing toluene diisocyanate (TDI) by means of the toluene diamine (TDA) phosgenation in the gaseous phase, with optional participation of an inert gas, relies on the fact that the main stream of phosgene supply (4) is divided into at least two streams: at least one outer shielding stream, which is fed so as to allow separation of the main area of the reaction from the reactor walls (9), while at least one main inner stream of phosgene (4) is fed to the main area of the reaction.
The device for obtaining toluene diisocyanate (TDI) by means of the toluene diamine (TDA) phosgenation in the gaseous phase, with the optional participation of an inert gas, comprises the reactor, the distributor (7) for introducing the raw materials in the gaseous phase to the reactor (9), wherein the distributor (7) is provided with central nozzles for supplying toluene diamine (TDA) and inner nozzles (3) for supplying phosgene, characterised by the fact that the reactor (9), which is shaped like divergent cylindrical tube, connected at the narrowest point (8) with the narrowest part of the distributor (7), which is shaped like convergent cylindrical tube, wherein in the central part of the distributor (7), inner nozzles (3) are located, supplying the main phosgene stream (4), outer annular nozzle (5) that supplies an additional phosgene stream and the main nozzle (2) that supplies toluene diamine (TDA).

## Description

The invention concerns a method for producing toluene diisocyanate (TDI) by means of the toluene diamine (TDA) phosgenation reaction in the gaseous phase and a device for obtaining toluene diisocyanate (TDI) by means of the toluene diamine (TDA) phosgenation reaction in the gaseous phase.

One of the well-known methods for producing toluene diisocyanate (TDI) is carrying out the phosgenation reaction of toluene diamine (TDA) in the gaseous phase. It is known that to achieve high efficiency and selectivity of the TDA phosgenation reaction in the gas phase, rapid mixing of phosgene with TDA is required, as well as avoiding backmixing, as described in many patent documents, such as EP 1319655, EP 0928785, EP 1275640, EP 0289840, EP 0749958, EP 1319655, US 2003/0216597, US 2004/0167354, US 2005/0113601, US 2005/0137417, WO 2010/010135.

The latter publication, WO 2010/010135, discloses a method based on the fact that the first reagent, i.e. amine in the form of vapour, is introduced to the outer part of a cylindrical distributor. The second component, i.e. phosgene, is fed to the distributor by the dosing tube located in the distributor axis, which is fed through the distributor side wall. The cylindrical distributor is connected to the divergent part of the reactor, the diffuser, which causes a favourable, in view of the process, increase in pressure and temperature.

So far, any attempts to accelerate the rate of mixing often cause an increase of backmixing intensity by increasing the intensity of circulation inside the reactor or enable the contact of the reaction mixture with the reactor walls, which reduces the selectivity and leads to the production of deposits. Moreover, when TDA is fed through centrally located nozzles, or by systems of nozzles directed parallel to the reactor axis, while phosgene is flowing through the reactor, increasing the intensity of mixing, requires raising the TDA vapour pressure, optionally diluted with an inert gas, which raises the temperature of TDA evaporation and may lead to partial thermal decomposition of TDA.

According to the invention, a method for preparing toluene diisocyanate (TDI) by conducting a toluene diamine (TDA) phosgenation reaction in the gas phase, with the optional participation of an inert gas, is based on the fact that the main stream of phosgene supply is divided into at least two streams, at least one external shielding stream, fed in a. manner that enables separation of the main area of the reaction from the walls of the reactor, while at least one main stream of phosgene is fed to the internal main area of the reaction.

Preferably, the reaction is carried out in a reactor that consists of a divergent cylindrical tube, in which the main stream of phosgene is fed through the inner nozzles located inside the main nozzle, through which the gas stream of (TDA) is fed, and the shielding stream of phosgene is fed through the annular nozzle between the main nozzle (TDA) mouth and the largest constriction point of the convergent part of the distributor, thus divergent part of the reactor, wherein the outlets of all streams are located in the largest narrowing of the convergent part of the distributor, thus divergent part of the reactor.

It is also preferable when the reaction is carried out with an excess of phosgene, wherein the main stream of phosgene provides at least a stoichiometrically equivalent amount in relation to the amount of (TDA) used.

It is also preferable when the reaction is carried out at a temperature from 200°C to 500°C, more preferably at a temperature from 300°C to 400°C.

It is advantageous when the reaction is carried out under a pressure of 300 to 500 kPa, more preferably under a pressure of 400 kPa.

It is also preferable when the average residence time in the reactor, active for the reaction, is maintained within the range from 0.5 sec. to 6 sec.

Another advantageous feature of the invention lies in the fact that the speed of phosgene in the inner nozzles is maintained within a range up to 30 m/s.

The device, according to the invention, for producing toluene diisocyanate (TDI) by conducting the toluene diamine (TDA) phosgenation reaction in the gas phase, with the optional participation of an inert gas, containing the reactor, the distributor for supplying raw materials in the gaseous phase to the reactor, wherein the distributor is provided with central nozzles introducing toluene diamine (TDA) and inner nozzles introducing phosgene, is characterised by the fact that the reactor has the shape of a divergent cylindrical tube, connected at the narrowest point with the narrowest part of the distributor, which is shaped like a convergent cylindrical tube, wherein in the central part of the distributor, inner nozzles are located, which provide the main stream of phosgene, as well as the outer annular nozzle, providing an additional stream of phosgene, and the main nozzle, providing the toluene diamine (TDA).

It is preferable when the minimum number of inner nozzles is one, and the maximum number is unlimited.

It is also preferable when the number of inner nozzles is three or multiples of three.

It is also advantageous when the inner nozzles mouths and the main nozzle mouth are located at the largest narrowing of the convergent part of the distributor and the divergent part of the reactor.

Another beneficial feature is when the cylindrical part of the distributor is provided with an element levelling the velocity at which phosgene is supplied through the annular nozzles, preferably a porous annular insert, a ring provided with a mesh or a perforated ring.

Another advantageous feature is that the inclination angle of the convergent part of the distributor is 19° to 21 °, more preferably 21 °.

Another advantageous feature is that the inclination angle of the divergent part of the distributor is 5° to 7°, more preferably 7°.

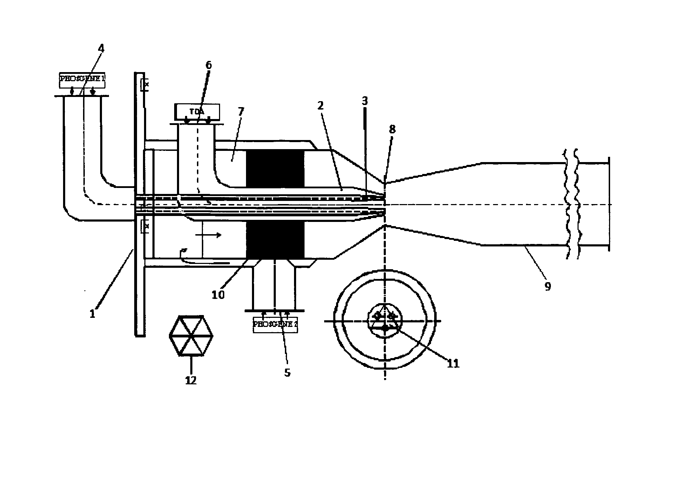

The embodiment of the method and device of the invention is shown in the drawing, in which Fig. 1 shows a longitudinal section of the reactor, where the distributor is presented schematically.

As demonstrated in the embodiment in Fig. 1, the device 1, according to the invention, wherein the method is carried out in accordance with the invention, comprises the reactor 9 connected to the distributor 7. The distributor 7 is provided for the task of preparing a mixture of components, properly prepared for the purpose of carrying out in the reactor 9 the reaction of separating reaction products from the toluene diamine (TDA) phosgenation reaction in the gas phase during the production of toluene diisocyanate (TDI) The distributor 7 is shaped like a convergent cylindrical tube, and the reactor 9 is shaped like a divergent cylindrical tube, wherein both of these two elements, 7 and 9, are connected to the largest narrowing point 8 of the convergent part of the distributor 7 and the divergent part of the reactor 9. The convergent part of the distributor 7 is inclined in relation to the longitudinal axis at an angle of 20°, and the divergent part is inclined in relation to the longitudinal axis at an angle of 7°.

Appropriate systems are placed inside the distributor 7 in order to prepare the implementation of the task mentioned beforehand of preparing the reaction components. In the part of the distributor 7 most distant from the largest narrowing point 8 and the reactor 9, the supply conduit of the main stream of phosgene 4 is placed, by means of which phosgene, involved in the reaction, is fed to the device 1. Inside the device, the main stream of phosgene 4 is directed to the inner nozzles 3. The inner nozzles 3 are placed inside the main nozzle 2 that feeds the toluene diamine (TDA) to the reaction site. The side wall of the cylindrical part of the distributor 7 is equipped with an annular nozzle 5, by means of which additional phosgene is fed to the distributor 7. This additional phosgene is fed between the inner walls of the distributor 7 and the outer wall of the main nozzle 2 to the point of the largest constriction 8 where the reactor 9 starts. In order to level out the velocity profile of the additional shielding phosgene, inside the distributor 7, a levelling element 10 in the form of a porous annular insert, a ring provided with a mesh or a perforated ring is placed.

In Fig. 1, a schematic arrangement of the three inner nozzles 11 inside the main nozzle 2 and inside the main distributor 7 at the largest narrowing point 8, in a cross section, is shown. The recommended number of nozzles is at least N=3. Multiples of this number are obtained according to the scheme 12 in Fig. 1, where the nozzles are placed in the middle of the sides of equilateral triangles formed in the manner shown in the Figure.

The method according to the invention relies on the fact that the substrates for TDA phosgenation and phosgene, optionally diluted by an inert gas, are fed separately into the reactor 9 through the distributor 7, which allows contact and initiates mixing of the reagents, wherein the mixing starts at the largest narrowing point in the convergent part of the distributor and the divergent part of the reactor 8. The reactants are fed in three main streams: firstly, in the main stream of phosgene 4 by means of inner nozzles 3, wherein the stream of phosgene is introduced under increased pressure relative to TDA vapour pressure; secondly, in an additional stream of phosgene introduced by means of outer annular nozzle 5, wherein this additional stream of phosgene shields the reactor walls 9 from direct contact with the reaction mixture and is involved in the reaction at the area close to the walls; thirdly, in a stream of TDA 6 introduced through the main nozzle 2 under relatively low pressure and optionally diluted with an inert gas. The reactor 9 is shaped like a divergent cylindrical tube with a low angle of divergence, wherein the divergent part is connected to the cylindrical portion of the reactor 9. Elements that provide the feeding of the reactor 9 are located in the distributor 7, whereas the stirring and the reaction take place in the divergent part and subsequent part of the cylindrical reactor 9.

The distributor 7 for components of the reaction mixture is provided with a convergent part, wherein the preferred position for the outlet of the feeding elements is located in the largest narrowing point 8 of the distributor 7 and thus the reactor 9. Through the main nozzle 2, TDA is fed, and the shielding phosgene is being fed by an annular nozzle 5 that forms an annular area around the central nozzle. Inside the central nozzle, the inner convergent nozzles 3 are located, with axes oriented parallel to the axis of the reactor 9, wherein the three inner nozzles 3 are preferred, and their multiples 12, with axes passing through the middle of the sides of an equilateral triangle inscribed in a circle, formed by the main nozzle mouth 2 and the outlet at the largest narrowing point 8 of the reactor 9, as shown in Figure 1. Inner nozzles 3 are used to introduce phosgene into the system at an elevated pressure, at an amount stoichiometrically equivalent or at the stoichiometric excess in relation to the amount of TDA that is being fed, and said phosgene is mixing and reacts at the core of the flow in the reactor 9. The process is turbulent in nature, and the measure of proper mixing intensity is a sufficiently low value of the mixing time constant in the inertial-diffusion region of the spectrum of turbulent concentration fluctuations.

The method for scaling up the process of conducting the phosgenation reaction involves enlarging the diameter of the reactor 9, the diameter of the largest narrowing size 8, the main nozzle 2 and inner nozzles 3, wherein it is preferable to maintain the stability of the outlet surface ratio of shielding phosgene and TDA, and increasing the number of inner nozzles 3 in accordance with the scheme presented in Figure 1, i.e. N = 1, N = 3, N = 13 - nozzles in the centre of a regular hexagon and in the middle of each side of the six triangles visible in Figure 1, item 12 - and combinations of such hexagons according to the scheme, that resembles a honeycomb. During the scaling up, it is preferable to increase the cross-sectional area of the reactor 9 in proportion to the load.

The solution according to the invention is different from the process described in the patent PL 181030, in which there is a division between the zone for mixing substrates and the reaction zones after completing the mixing. According to the invention, the mixing and the reactions take place in the whole space of the reactor, starting from the outlet of the feeding elements at the largest narrowing point of the reactor.

### Comparative Example

In the reactor 9 with a diameter of 100 mm in the cylindrical part, the diameter of the distributor 7 equal to 130 mm, the length of the convergent part of the distributor 7 equal to 157.5 mm and the length of the divergent part of the reactor 9 equal to 280 mm, TDA phosgenation was carried out with a reactor 9 load equal to 50 kg/h; TDA, double excess of phosgene and TDA diluted with nitrogen in a molar ratio of 2 parts N₂: 1 part TDA, resulting in the overall reactor 9 load being 195 kg/h. The main stream of phosgene was fed through three inner nozzles 3 with a tube diameter of 8 mm, mouth diameter of 6 mm and a length of constriction of 30 mm. TDA was fed through the main nozzle 2 with a tube diameter of 80 mm, mouth diameter equal to 56 mm and a length of constriction of 70 mm. The shielding stream of phosgene was introduced through the annular area 10 between the main nozzle 2 mouth, that supplies TDA, and the largest narrowing point 8 in the convergent part of distributor 7, and thus the divergent parts of the reactor 9, having a diameter of 76 mm. The exit point of the inner nozzle 3 mouths of the main stream of phosgene and the main nozzle of the TDA nozzle are located in the largest narrowing point 8 of the reactor 9, thus the largest narrowing point 8 of the distributor 7. The shielding stream of phosgene was fed through the annular outer nozzle 5. The overall flow rate of phosgene was 122 kg/h, wherein it was supplied in the proportions 2/3 through the inner nozzles 3 and 1/3 through the outer annular nozzle 5. The inlet temperature was equal to 573.15 K, while at the outlet of a 1 meter long cylindrical reactor 9, this temperature was 649 K. Velocity at the mouth of the inner nozzles 3 was 34 m/s at an inlet pressure of 4.0 bar of phosgene and a pressure of 3.91 bar of amine.

At the mixture leaving the reactor 9, total completion of the reaction of TDA was achieved, with 31.5% by weight of TDI, about 26% by weight of phosgene, about 4% of the carbamoyl chlorides, about 0.04% of hydrochloride TDA deposits and derivatives of TDA destruction, 0.012% of ammonium chloride, less than 0.02% of urea derivatives and 25% of hydrogen chloride.

The method of supplying the reactants prevented the formation of strong vortices in circulation and thus eliminated the backmixing, stabilised the flow and ensured an increase in pressure in the reaction area.

Streams of phosgene were used, fed through the inner nozzles 3, located in the large main nozzle 2 for feeding TDA, to generate the partial vacuum that facilitates the feeding of reactor 9 in TDA vapours, without the need to feed TDA under a significantly higher pressure. Therefore, as a result, the need to increase the temperature of TDA evaporation was eliminated.

Legend:
1. Device
2. Main nozzle
3. Inner nozzles
4. Main stream of phosgene
5. Annular nozzle
6. TDA stream
7. Distributor
8. Largest narrowing
9. Reactor
10. Levelling element
11. Arrangement of inner nozzles
12. Scheme for enlarging the number of inner nozzles

## Claims

1. A method for producing toluene diisocyanate (TDI) by means of the toluene diamine (TDA) phosgenation in the gaseous phase, with optional participation of an inert gas, **characterised in that** the main stream of phosgene supply (4) is divided into at least two streams: at least one outer shielding stream, which is fed so as to allow separation of the main area of the reaction from the reactor walls (9), while at least one main inner stream of phosgene (4) is fed to the main area of the reaction.

2. The method according to claim 1, **characterised in that** the reaction is carried out in a reactor (9), which consists of a divergent cylindrical tube, in which the main inner stream of phosgene (4) is fed through the inner convergent nozzles (3), located inside the main nozzle (2) through which the gas stream of TDA (6) is fed, and the shielding stream of phosgene is fed through the annular nozzle (5), between the main nozzle (TDA) (2) mouth, and the largest narrowing point (8) of the convergent part of the distributor (7), thus divergent part of the reactor (9), wherein the outlets of all streams are located in largest narrowing (8) of the convergent part of the distributor (7), thus divergent part of the reactor (9).

3. The method according to claim 1, **characterised in that** the reaction is carried out with an excess of phosgene, wherein the main stream of phosgene (4) provides at least a stoichiometrically equivalent amount in relation to the amount of (TDA).

4. The method according to claim 1, **characterised in that** the reaction is carried out at a temperature from 200° to 500°C.

5. The method according to claim 4, **characterised in that** the reaction is carried out at a temperature from 300° to 400°C.

6. The method according to claim 1, **characterised in that** the reaction is carried out under a pressure of 300 to 500 kPa.

7. The method according to claim 6, **characterised in that** the reaction is carried out under a pressure of 400 kPa.

8. The method according to claim 1, **characterised in that** the average residence time in the reactor, active for the reaction, is maintained within the range from 0,5 sec. to 6 sec.

9. The method according to claim 1, **characterised in that** the speed of phosgene in the inner nozzles (3) is maintained within a range up to 30 m/sec.

10. A device for obtaining toluene diisocyanate (TDI) by means of the toluene diamine (TDA) phosgenation in the gaseous phase, with the optional participation of an inert gas, that comprises the reactor, the distributor for introducing the raw materials in the gaseous phase to the reactor, wherein the distributor is provided with central nozzles for supplying toluene diamine (TDA) and inner nozzles for supplying phosgene, **characterised in that** the reactor (9) is shaped like divergent cylindrical tube that is connected at the narrowest point (8) with the narrowest part of the distributor (7) which is shaped like convergent cylindrical tube, wherein in the central part of the distributor (7) inner nozzles (3) are located, supplying the main phosgene stream (4), as well as the outer annular nozzle (5) that supplies an additional phosgene stream and the main nozzle (2) that supplies toluene diamine (TDA).

11. The device according to claim 10, **characterised in that** the minimum number of inner nozzles is one, and the maximum number is unlimited.

12. The device according to claim 11, **characterised in that** the number of inner convergent nozzles (3) is three or multiples of three.

13. The device according to claim 10, **characterised in that** the inner convergent nozzles (3) mouths and the main nozzle (2) mouth are located at the largest narrowing (8) of the convergent part of the distributor (7) and the divergent part of the reactor (9).

14. The device according to claim 10, **characterised in that** the cylindrical part of the distributor (7) is provided with a levelling element (10) that levels the velocity at which phosgene is supplied through the annular nozzle (5).

15. The device according to claim 12, **characterised in that** the levelling element (10) is a porous annular insert.

16. The device according to claim 12, **characterised in that** the levelling element (10) is a ring provided with a mesh or a perforated ring.

17. The device according to claim 10, **characterised in that** the inclination angle of the convergent part of the distributor (7) is 19° to 21°.

18. The device according to claim 14, **characterised in that** the inclination angle of the convergent part of the distributor (7) is 20°.

19. The device according to claim 10, **characterised in that** the inclination angle of the divergent part of the reactor (9) is 5° to 7°.

20. The device according to claim 16, **characterised in that** the inclination angle of the divergent part of the reactor (9) is 7°.
